# EUROPEAN PATENT SPECIFICATION

(11) **EP 1 880 739 B1**
(45) Date of publication and mention of the grant of the patent: **13.09.2017**
(21) Application number: 05028319.1
(22) Date of filing: 23.12.2005
(51) Int. Cl.: A61L 27/12, A61L 27/22, A61L 27/54

(54) **Process for the immobilization of proteins on an implant**
Verfahren zur Immobilisierung von Proteinen auf Implantaten
Procédé d'immobilisation de protéines sur des implants

(43) Date of publication of application: 23.01.2008
(73) Proprietor: Jennissen, Herbert P., Prof. Dr., 50858 Köln (DE)
(72) Inventor: Jennissen, Herbert, P., Prof. Dr., 50858 Köln (DE); Laub, Marcus, Dr., 45134 Essen (DE); Chatzinikolaidou, Maria, Dr., 45147 Essen (DE); Zurlinden, Kristin, Dr., 45721 Haltern am See (DE)
(74) Representative: Nobbe, Matthias

(56) References cited:
- WO-A-96/09078
- WO-A-99/26674
- JENNISSEN H P ET AL: "BIOCOATING OF IMPLANTS WITH MEDIATOR MOLECULES: SURFACE ENHANCEMENT OF METALS BY TREATMENT WITH CHROMOSULFURIC ACID" MATERIALWISSENSCHAFT UND WERKSTOFFTECHNIK, VCH VERLAGSGESELLSCHAFT, WEINHEIM, DE, vol. 30, no. 12, 1999, pages 838-845, XP008019584 ISSN: 0933-5137

## Description

The present invention refers to the chemical functionalisation of surfaces, in particular ceramic materials, particularly hydroxyapatite and the use of tensides for improving the absorption characteristics of metallic or ceramic surfaces.

Hydroxyapatite (HAP) has a great potential as a bone replacement material because of its similarity to the crystal structure of inorganic matrix of bone. Several years ago we showed that porous hydroxyapatite ceramics (Endobon, Merck) can be covalently modified for the immobilization of proteins [Jennissen, H.P. (1999) PCT Patent WO9926674A2] opening the possibility for the immobilization of growth factors and morphogens. Application of this technique to the immobilization of recombinant human bone morphogenetic protein 2 (rhBMP-2) on hydroxyapatite would be of high medical interest, because of the decisive role of bone morphogenetic proteins in bone development and osteogenesis. Here, it will be shown that the model protein ubiquitin and rhBMP2 can be immobilized non-covalently and covalently after chemical modification of a hydroxyapatite ceramic surface (Bio-Oss^{®} Cancellous Block, Geistlich, = HAP-wafers). It could be shown that only small amounts of protein are adsorbed to non-functionalized HAP surfaces (control). In contrast ubiquitin and rhBMP-2 can be very efficiently immobilized non-covalently (up to 2.4 mg protein/g HAP) and covalently (up to 9.7 mg protein/g HAP) on porous HAP-wafers. In desorption experiments it is shown that especially the bound rhBMP-2 is slowly released making such surfaces applicable as long term drug delivery devices for enhancing bone growth and osteointegration of implant materials.

### State of the Art

Bone is composed to ∼ 60% of hydroxyapatite (HAP, see[1]), which in its crystalline form has the chemical composition Ca₁₀(PO₄)₆(OH)₂ (see [2-4]). Hydroxyapatite has a very low solubility product of 2.34 x 10⁻⁵⁹ with a calculated dissociation constant of ∼0.44 µM [5]. Theoretical studies indicate that several layers of water molecules are bound to the crystals but that the hydroxyl ions at the apatite surface cannot be protonated [6]. On the other hand the phosphate oxygens appear susceptible to protonation [6]. Based on the known structural composition of hydroxyapatite similarities to glass and other ceramics we therefore reasoned that a reaction with silanes should be possible. Previously we had shown that the amount of ¹²⁵I-ubiquitin nonspecifically bound to the porous hydroxyapatite ceramic Endobon [7] (Merck AG Darmstadt, density 1.289 g/cm³, ∼100% HAP [1]) can be increased from 6 µg/g to 24-30 µg/g by covalently linking it via a reaction with 3-aminopropyltriethoxysilane (APS) followed by carbonyldiimidazole (CDI) [8]. A similar reaction scheme has been employed by our group for several years for coupling rhBMP-2 to titanium surfaces [8,9]. In this connection the immobilization of rhBMP-2 on hydroxyapatite would be of high medical interest, because hydroxyapatites either in the form of individual granules or blocks [1] or as plasma spray coatings on implants (see [10]) are widely employed in the therapy of bone defects and bone disease. The immobilized rhBMP-2 could lead to an enhancement of bone growth and replacement of the artificial hydroxyapatite by endogenous bone. Here, the inventors will show that BMP-2 can be chemically immobilized on porous HAP-wafers (Bio-Oss^{®} Cancellous Block i.e. Orthoss^{®}, Geistlich) and that this mediator is slowly released from the surface with the potential of osteoinduction.

The present invention is characterized by the findings that the use of amphiphilic tensides, can improve the surface absorptions characteristics of materials which can serve a implant materials, made from metals, alloys, ceramic materials and combinations thereof.

Amphiphilic is a chemical compound possessing both hydrophilic and hydrophobic nature. A compound with such properties is called amphiphilic which is synonymous to amphipathic. Molecules of amphiphilic compounds have hydrophobic (usually of hydrocarbon nature) and hydrophilic (represented by either ionic or uncharged polar functional groups) structural regions. As a result of having both hydrophobic and hydrophilic structural regions, some amphiphilic compounds may dissolve in water and to some extent - in non-polar organic solvents.

Many amphiphilic compounds strongly interact with biological membranes by insertion of hydrophobic part into the lipid membrane, while exposing the hydrophilic part to the aqueous medium. When placed in an immiscible biphasic system consisting of aqueous and hydrophobic solvent the amphiphilic compound will partition between the two phases. The balance between hydrophobic and hydrophilic natures defines the extent of partitioning. Surfactants are an example group of amphiphilic compounds. In the present invention, amphiphilic compounds are bile acid derivatives having a zwitterionic character, in particular, CHAPS 3-[(3-Cholamidopropyl)-dimethylammonio]-propansulfonate.

The inventors found out that the chemisorption of biologically active molecules on surfaces, in particular hydrophilic surfaces, can be largely improved when, in the step of treating the surface with such bioactive molecules such as BMP, particularly BMP-2, an amphiphilic tenside like CHAPS 3-[(3-Cholamidopropyl)-dimethylammonio]-propansulfonate or similar bile acid derivatives having a zwitterionic character or a mixture thereof is present in the treatment solution. The surfaces preferably comprise surfaces of metallic or ceramic implants like titan implants, ceramic implants like hydroxyapatite, or combinations thereof and the like. The biologically active molecules are selected from growth factors of the class of TGF proteines, in particular BMP, more preferably BMP-2, vascular growth factors like VEGF or angiotropin, ubiquitin, antibiotics or mixtures thereof.

### MATERIALS AND METHODS

### Materials

3-Aminopropyltriethoxysilane and ubiquitin were purchased from Sigma-Aldrich Chemie GmbH, 82024 Taufkirchen, Germany. Carbonyldiimidazole was obtained from Acros Organics (Fisher Scientific GmbH, 58239 Schwerte, Germany). Dry acetone was obtained from Merck (VWR International GmbH, 64295 Darmstadt, Germany). As bone replacement material Bio-Oss^{®} Cancellous Blocks i.e. Orthoss^{®} Spongiosa Blocks, obtained from Geistlich Pharma GmbH (CH-6110 Wolhusen, Switzerland), were used. For experiments the blocks were cut into small wafers (approx. 10 x 10 x 2 mm, = **HAP-wafers**). The material consists of 93.6 % hydroxyapatite (HAP), 3.4 % CaCO₃ and 3.0 % water [1]. The inner specific surface area as computed from nitrogen isotherms is 79.7 m²/g (i.e. 7.9 x 10⁵ cm²/g) with a porosity of 60% and a pore range of 2-50 nm having a maximum incidence at 10 nm [11]. Because of their fragility the size and weight of the HAP-wafers varied considerably: 0.178 g ± 0,065 g (n = 47). All other chemicals were of the highest available or analytical grade. Water was prepared by distillation of deinonized water and then passing it through a MilliQ-System (Millipore, D-65760 Eschborn, Germany).

### Used Methods

### Preparation and testing of proteins

Radioactively labelled ubiquitin (¹²⁵I-CT-ubiquitin, ¹²⁵I-ubiquitin) was prepared by the chloramine-T method as previously described [9]. The biological activity of ubiquitin is not decreased but enhanced by this iodination procedure [12]. Recombinant human bone morphogenetic protein 2 (rhBMP-2) was prepared as described previously [9]. Escherichia coli BL21(DE3), BL21(DE3)pLYs (Novagen, Madison, WI, USA) and Rosetta 2(DE3) (Novagen, Madison, WI, USA) were used as hosts for expression vector pet24c (Calbiochem-Novobiochem GmbH, Bad Soden, Germany) [13]. The mature rhBMP-2 coding sequence (114 amino acids) is preceded by GCC which codes for serine. N-terminal sequence analysis using automated Edman degradation (NAPS Unit, University of British Columbia, Canada) confirmed the existence of this modification. Briefly E. coli cells were centrifuged and washed with 50 mM Tris, 100 mM NaCl, pH 8. Inclusion bodies were extracted from frozen cell pellets (-80 °C) by homogenization in 50 mM Tris, 5 mM DTT, 1 mM EDTA, pH 8 using an Ultra Turrax (Janke & Kunkel, Stauffen, Germany) and subsequent centrifugation. Pellets were stored frozen at -80 °C. Inclusion bodies were solubilized in 10 mM Tris, 50 mM mercaptoethanol, 6 M guanidinium hydrochloride, pH 7.6. Refolding was achieved by the glutathione method. Refolded rhBMP-2 was pressure concentrated using an Amicon cell (Amicon, Witten, Germany) i followed by gelfiltration on Sepharose 6 pg. Fractions containing rhBMP-2 were identified by 17 % SDS polyacrylamide gel electrophoresis (SDS-PAGE), pooled and applied to affinity chromatography on Heparin Sepharose 6 Fastflow (Amersham, Freiburg, Germany) [14]. The column was eluted with a linear gradient (0.15-1 M NaCl). Fractions were analyzed by 17 % SDS-PAGE. Alternatively heparin affinity chromatography in the presence of 4 M urea [14] was used as a capture step followed by gelfiltration on Superose 6pg.

The biological activity of rhBMP-2 was tested with MC3T3-E1 cells by the activation of the de novo synthesis of alkaline phosphatase [15]. Radioactive labelled rhBMP-2 (¹²⁵I-CT-rhBMP-2, ¹²⁵I-rhBMP-2) was prepared by a modified chloramine-T method [16]. The biological activity of rhBMP-2 remains unchanged after iodination (unpublished) indicating that there is no major change in conformation. The specific radioactivity was adjusted by dilution with cold-labelled iodinated CT-proteins.

### Surface functionalization with 3-aminopropyltriethoxysilane [8]

The HAP wafers (approx. 10 x 10 x 2 mm) were reacted with 3-aminopropyltriethoxysilane (APS) in a teflon holder in 47.5 ml toluene to which 2.5 ml APS (i.e. 5 % vol/vol) had been added under inert gas [8]. The closed system was boiled under reflux and stirring for 3.5 hours. The wafers were then washed 3 times in 10 ml trichloromethane, acetone and methanol and air dried (HAP-APS wafers).

### Surface activation with carbonyldiimidazole

For covalent protein coupling the HAP-APS wafers were activated with 1,1'-carbonyldiimidazole (CDI) [8]. The wafers were reacted in a teflon holder with a solution of 2.5 g CDI in 50 ml dry acetone under inert gas for 3.5 hours (HAP-APS-CDI wafers). The CDI-activated wafers were then washed 3 times in 10 ml acetone and water.

### Coupling of proteins

The procedures are similar to those employed for the immobilization of proteins on metal surfaces [9,16]. For immobilization of ubiquitin the washed wafers (3 x HAP as control, 3 x HAP-APS, 3 x HAP-APS-CDI) were incubated for 15 hours at room temperature in a solution of 50 mM sodium phosphate pH 10.0 (buffer A) containing ubiquitin (0.1 - 3.5 mg /ml). The amount of bound protein is determined by using ¹²⁵I-CT-ubiquitin. The specific radioactivity was ca. 10 µCi/mg. After incubation the wafers were rinsed 3 times in 5 ml buffer A and counted in a γ-counter. The incubation of rhBMP-2 proceeds the same way as with ubiquitin and takes place in 0.1 - 0.3 mg/ml protein solution in 125 mM borate, 5 mM CHAPS, 0.066 % SDS, pH 10.0 (buffer B). The concentrations given are initial concentrations. On addition of the HAP-wafer the initial concentrations decreased in the mean for ubiquitin on HAP-APS-CDI by 64% on HAP-APS by 21% and on HAP by 2%. In the case of rhBMP-2 the initial concentrations decreased in the mean on HAP-APS-CDI by 65% on HAP-APS by 33% and on HAP by 8%. The experiments were performed in triplicate (n = 3) except in the case of ubiquitin on HAP-APS-CDI 0.3 mg/ml, 3.5 mg/ml, on HAP-APS 0.3 mg/ml, 3.5 mg/ml and on HAP 0.3 mg/ml where n = 2 respectively.

### Desorption experiments

The desorption procedures are similar to those employed for the desorption of proteins from metal surfaces [16]. After adsorption of ubiquitin (see above) and its measurement in the γ-counter the wafers were transferred to 5 ml buffer A for desorption measurements. At specified times (initially every 24 hours) the wafers were taken out, washed in 3 x 5 ml buffer A and measured in the γ-counter. They were then resuspended in 5 ml fresh buffer A for the next period of desorption. This regime was continued for up to 50 days. The desorption experiment with immobilized rhBMP-2 proceeds the same way as with ubiquitin but takes place in buffer B. Desorption was observed for up to 25 days. In similar experiments (similar half-lives) with titanium miniplates (Chatzinikolaidou & Jennissen in preparation) the kinetics were checked for readsorption. Within the above time and buffer volume regimens no readsorption was detected for the miniplates, which is also surmised for the wafers. The calculated half-lives of desorption were corrected for the spontaneous decay of ¹²⁵I (t_{1/2} = 60 d).

### Cell culture

Mouse calvaria osteoblast MC3T3-E1 cells (Deutsche Sammlung von Mikroorganismen und Zellkulturen. D-38124 Braunschweig; passages 4 - 12) were grown in 50 ml flasks using α-modified Eagles Medium (α-MEM), supplemented with glutamine (2 mM), penicillin (50 I.U./ml), streptomycin (50 µg/ml) and either 1 % or 10 % (v/v) fetal calf serum (FCS) in a humidified atmosphere and 5 % CO₂ at 37 °C in a cell incubator (Hera Cell, Heraeus) (see [15]). Confluent cells were washed with Dulbeccos phosphate buffer saline without calcium and manganese (DPBS, 137 mM NaCl, 8.1 mM Na₂HPO₄, 2.7 mM KCl, 1.5 mM KH₂PO₄, pH 7.4, PAA, Linz, Austria) and passaged after trypsination (0.25 % trypsin in 1 mM EDTA) for 30 - 60 s, seeded at 50 - 60 % confluence and allowed to grow for 4 - 5 days before the next passage.

### Biological activity measurement of soluble rhBMP-2

The bioassay of BMP-2 activity (MC3T3-cell ALP-induction test) is based on the induction of alkaline phophatase (ALP) in MC3T3-E1 cells by BMP-2 [15]. 5 x 10⁵ fresh trypsinized cells were seeded on HAP pieces placed in the wells of a 48 well plate using α-MEM containing 10 % FCS. After 24 hours the medium of the now confluent cells was replaced by a α-MEM and 20 nM rhBMP-2 containing 1 % FCS. The cells were incubated in a humidified atmosphere and 5 % CO₂ at 37 °C in a cell incubator. After 3 days the cells were carefully rinsed three times with Dulbeccos phosphate buffer and fixed with 2 % paraformaldehyde for 10 min. After fixative was removed by the three brief washes with phosphate buffer, cell membranes were permeabilized by washing with DPBS/Tween 20 0.2 % (v/v) and were then rinsed with phosphate buffer. The endogenous phosphatase detection kit ELF-97 (Substrate: 2-(5'-chloro-2'-phosphoryloxyphenyl)-6-chloro-4-(3H)-quinazolinone; Molecular Probes, Inc., Oregon, USA) was employed in the cell culture experiments according to the manufacturer's instruction (see [17]). The resulting product forms an intensely fluorescent yellow-green precipitate at the site of enzymatic activity. Cell culture reagents were from Gibco (Invitrogen GmbH, Karlsruhe, Germany). 5 - 10 minutes after the filtered alkaline phosphatase substrate solution was added, the color development the reaction was stopped by addition of 500 µl levamisol hydrochloride (1-2,3,5,6-tetrahydro-6-phenyl-imidazole(2,1-b)thiazole, Synopharm GmbH, Barsbüttel, Germany). Cells were rinsed with phosphate buffer and the ALP-activity of BMP-2 stimulated cells was monitored in a fluorescence microscope (Nikon Eclipse E 400, Nikon GmbH, Düsseldorf, Germany), using the appropriate optical filters for the visualization of the fluorescent substrate (excitation wave length: 345 nm, emission wave length: 530 nm). After that DAPI (4',6-Diamidino-2-phenylindole · 2 HCl) was added to the cell culture experiments the nuclei could be visualized. The probes were digitally imaged in the fluorescence microscope (excitation wave length: 330 nm, emission wave length 400 nm).

### Curve fitting and statistics.

For curve fitting and statistics of the kinetic data the PC program Prism 4 (Graph Pad Software Inc., San Diego, CA) was employed. In all cases the results are given as mean values ± standard deviation (S.D.).

### Brief description of the drawings

***Figure 1*** ***- Immobilization of ¹²⁵I-Ubiquitin on HAP-Wafers.***
   ¹²⁵I-Ubiquitin was adsorbed to modified and non-modified HAP wafers in the amounts indicated in **Table 1.** The amount of bound protein depends on the protein concentration of the incubation solution (0.1 - 3.5 mg/ml). For further details see Table 1, Methods and the text.
**Figure 2** **- Exponential Release of ¹²⁵I-Ubiquitin from HAP-Wafers.**
   The data are shown in a semilogarithmic plot in agreement with first-order kinetics. The release of covalently linked ¹²⁵I-ubiquitin (HAP-APS-CDI) occurs monophasically. The release of non-covalently immobilized ¹²⁵I-ubiquitin (HAP, HAP-APS) occurs as a two-phase exponential release. The half-lives are given for the fast phase 1 and the slow phase 2 respectively. After adsorption of ¹²⁵I-ubiquitin and measurement in the γ-counter the wafers were transferred to 5 ml buffer A for desorption measurements. At specified times the wafers were taken out, washed in 3 x 5 ml buffer A and measured in the γ-counter. They were then resuspended in 5 ml fresh buffer A for the next period of desorption. For further details see Methods, Table 3 and the text.

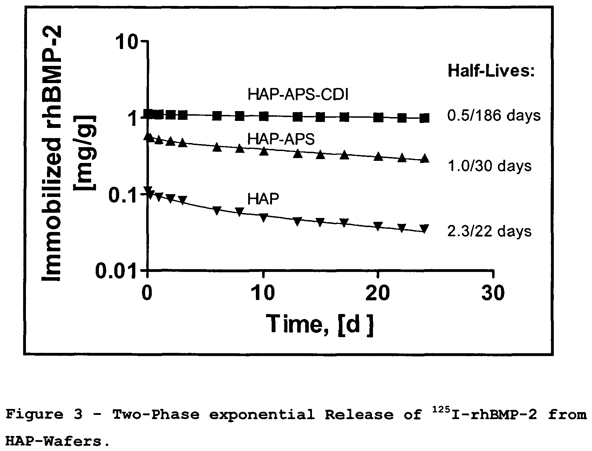

**Figure 3** **- Two-Phase exponential Release of ¹²⁵I-rhBMP-2 from HAP-Wafers.**
   The data are shown in a semilogarithmic plot in agreement with first-order kinetics. In all cases the release of immobilized ¹²⁵I-rhBMP-2 occurs in the form of a two-phase exponential function. The half-lives are given for the fast phase 1 and the slow phase 2 respectively. After adsorption of ¹²⁵I-rhBMP-2 and measurement in the γ-counter the wafers were transferred to 5 ml buffer B for desorption measurements. At specified times the wafers were taken out, washed in 3 x 5 ml buffer B and measured in the γ-counter. They were then resuspended in 5 ml fresh buffer B for the next period of desorption. For further details see Methods, Table 3 and the text.

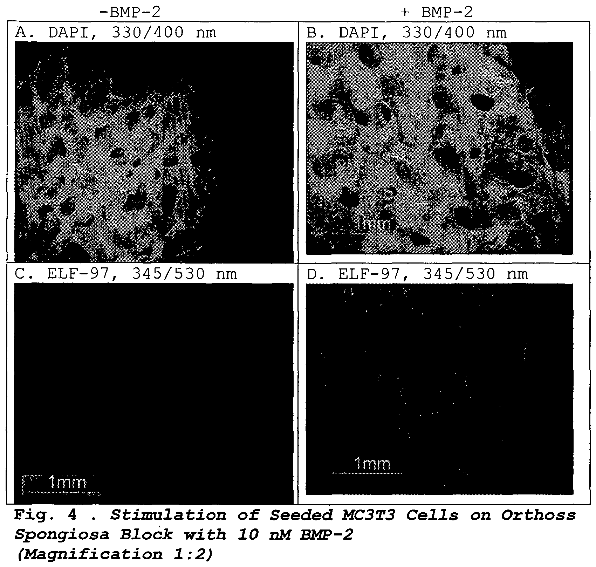

**Fig. 4** **- *Stimulation of Seeded MC3T3-E1 Cells on HAP-wafers with soluble BMP-2***
   The bioassay of BMP-2 activity (MC3T3-cell ALP-induction test) is based on the induction of alkaline phosphatase (ALP) in MC3T3-E1 cells by BMP-2. 5 x 10⁵ fresh trypsinized cells were seeded on HAP pieces placed in the wells using α-MEM containing 10 % FCS. After 24 hours the medium of the now confluent cells was replaced by a α-MEM and 20 nM rhBMP-2 containing 1 % FCS. After 3 d the cells were fixed and cell membranes were permeabilized. The endogenous phosphatase detection kit ELF-97 was employed in the cell culture experiments. The resulting product forms an intensely fluorescent yellow-green precipitate at the site of enzymatic activity. The ALP-activity of BMP-2 stimulated cells was monitored in a fluorescence microscope (magnification 1:2). After that DAPI was added to the cell culture experiments to demonstrate the cell nuclei and the probes were then monitored again in the fluorescence microscope. For further details see Methods.

**A:** DAPI and ELF-97 added to HAP with fixed cells and without BMP-2; **B:** DAPI and ELF-97 added to HAP with fixed cells and with BMP-2; **C:** ELF-97 added to HAP without fixed cells and without BMP-2; **D:** ELF-97 added to HAP with fixed cells and with BMP-2.

### Immobilization of Ubiquitin and rhBMP-2 on Porous Hydroxyapatite Wafers

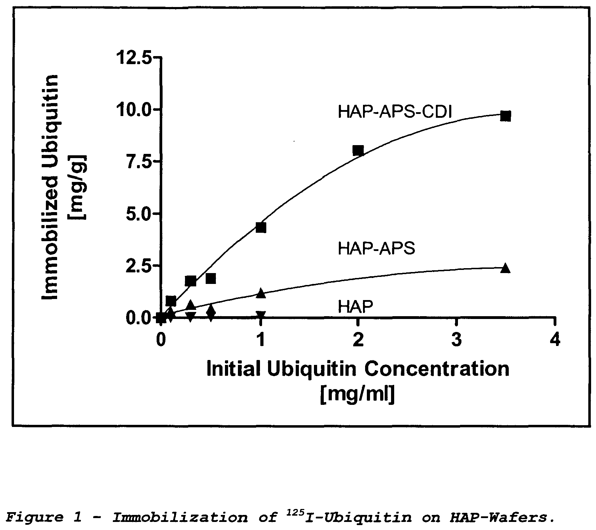

The immobilization of ¹²⁵I-ubiquitin on HAP-wafers is shown in **Fig. 1** after incubation of the functionalized wafers with ¹²⁵I-ubiquitin at room temperature for 15 hours. In the case of the APS functionalization (HAP-APS wafers) the surface is covered by aminopropyl groups. These groups lead to a hydrophobic adsorption of ¹²⁵I-ubiquitin so that the immobilization of ¹²⁵I-ubiquitin in this manner corresponds to an adsorption isotherm. At a free concentration of 3.5 mg/ml 2.4 mg/g of ubiquitin is adsorbed. For covalent linkage of ¹²⁵I-ubiquitin the primary amino group of the propel residue was activated with carbonyldiimidazole (HAP-APS-CDI wafers) and then reacted with ¹²⁵I-ubiquitin. Under these conditions 4-fold higher values up to 9.7 mg/g are immobilized. Non-specific adsorption on the unmodified control wafer (HAP) is negligible. Detailed data are given in **Table 1,** which also shows the values obtained by considering the surface area of the wafers. It can be seen that only 3-12 ng of ¹²⁵I-ubiquitin is immobilized per cm² surface area. These very low values can be explained by the extremely high surface area of 79.7 m²/g as measured by means of nitrogen isotherms. Since protein molecules are much larger than nitrogen they are excluded from a large proportion of the pores 2-50 nm in diameter. Thus pores with diameters smaller than 5-10 nm will be unavailable to ubiquitin and rhBMP-2 for immobilization. Therefore in the following all of the data will be normalized to the dry weight of the HAP-wafer. **Table 1** demonstrates that very large amounts of protein (∼ 10 mg/g) can be immobilized on HAP-wafers by covalent linkage.

In **Table 2** the immobilization of ¹²⁵I-rhBMP-2 HAP-wafers under similar conditions as for ¹²⁵I-ubiquitin is shown. In this case the concentration range of free ¹²⁵I-rhBMP-2 in the incubation mixture was in a lower range of 0.1-0.3 mg/ml. The amount of ¹²⁵I-rhBMP-2 adsorbed (HAP-APS wafers) at 0.3 mg/ml i.e. 0.59 mg/g wafer corresponds to similar values obtained for ubiquitin at 0.5 mg/ml (**Table 1**). Covalent immobilization (HAP-APS-CDI wafers) of ¹²⁵I-rhBMP-2 at 0.3 mg/ml reached 1.1 mg/g wafer, which is also similar to the amount of ubiquitin immobilized at 0.5 mg/ml (compare **Table 1**). Thus the similarity of the immobilization values of ¹²⁵I-ubiquitin and ¹²⁵I-rhBMP-2 indicate that the very high protein binding capacities (10 mg/g wafer) obtained with ¹²⁵I-ubiquitin can also be obtained with ¹²⁵I-rhBMP-2.

### Release Kinetics of Ubiquitin and rhBMP-2 from Porous Hydroxyapatite Wafers

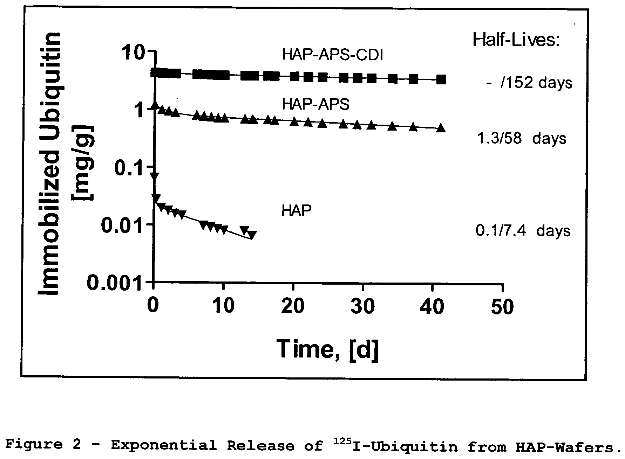

Next it was of interest to learn if the proteins can be released form the HAP-wafer surface. The desorption of immobilized ¹²⁵I-ubiquitin from the wafers is shown in **Fig. 2****.** The release is a first-order decay, either monophasic (HAP-APS-CDI) or biphasic (HAP, HAP-APS). ¹²⁵I-ubiquitin adsorbed non-specifically to HAP is released with the highest rates yielding half-lives of 0.1.to 7.4 days. In addition only very low amounts of ubiquitin are bound to this control. On the other hand ¹²⁵I-ubiquitin adsorbed to aminopropyl groups on HAP-APS is released in the major slow phase with a half-life of 58 days. At the beginning of desorption there is a slight burst of ¹²⁵I-ubiquitin release with a half-life of 1.3 days. Covalently immobilized ¹²⁵I-ubiquitin on (HAP-APS-CDI) is released in a single exponential phase with an extremely long half-life of 152 days. Even this low release rate is unexpected for a covalent linkage. It is probably due to the slow hydrolysis of one of the linking bonds. Similarly ¹²⁵I-rhBMP-2 is also released according to two-phase exponential functions from the wafers (**Fig. 3**). Here again ¹²⁵I-rhBMP-2 adsorbed to the control (HAP) is release with the highest rates but much more slowly (half-life 22 days) than ubiquitin (see **Fig. 2**). Hydrophobically adsorbed ¹²⁵I-rhBMP-2 on the aminopropyl residues is released with a burst phase half life of one day and a slow release phase with a half-life of 30 days. Covalently linked ¹²⁵I-rhBMP-2 is released with the extreme half-life of 186 days. There is a short burst phase with a half-life of 0.5 days which is probably due to non-covalently adsorbed ¹²⁵I-rhBMP-2.

The kinetic constants of the release kinetics of Fig. 2 & 3 are shown in **Table 3.** In general two-phase kinetics are observed (see above). The first order dissociation rate constants for the first fast phase lie in the range of 0.3-7.5 days⁻¹. In contrast the slow phases have first order dissociation rate constants in the range of 10⁻² to 10⁻³ days⁻¹.

Finally the kinetics of Figs. 2 & 3 allow the calculation of sustained releases of ¹²⁵I-ubiquitin and ¹²⁵I-rhBMP-2 from the HAP-wafers (see **Table 4**). These release rates were calculated for the first half-life of the slow release phase. In the case of non-covalently adsorbed ¹²⁵I-ubiquitin to HAP-APS the mean sustained release rate is 10 µg/g · day. This amount can (in the mean) be theoretically released for about 58 days. A similar amount of ubiquitin (i.e. 14 µg) can be released per day from the covalently immobilized ubiquitin - in this case - for 152 days. Similarly an amount of ca. 10 µg/g · day of ¹²⁵I-rhBMP-2 is released from HAP-APS surface. The duration of this release is 30 days. In this case the amount of ¹²⁵I-rhBMP-2 released from the covalently linked species (HAP-APS-CDI) is threefold lower than from the HAP-APS surface. For both proteins the controls are much lower than chemically immobilized species.

The question which now arises is whether the amounts of ¹²⁵I-rhBMP-2 released from the various surfaces is capable of stimulating bone growth. Specifically, are released amounts of ∼10 µg/g · day sufficient to attract bone precursor cells. It appears that this is indeed possible. We have previously shown [18] that 1 µg of rhBMP-2 immobilized on a miniplate is capable of bone induction in an ectopic bone healing model in the rabbit. In addition the amount of released ¹²⁵I-rhBMP-2 may be increased in the body as the hydroxyapatite is degraded and replaced by endogenous bone. This is an important question to be answered in future work.

### In Vitro Testing

In this connection it is of high importance to devise an *in vitro* test for biocoated porous HAP ceramics. A Pilot experiment to this effect is shown in **Fig. 4****.** MC3T3-E1 cells were therefore seeded on HAP-wafers and stimulated with rhBMP-2. Surprisingly at the fluorescence wave lengths 330 nm excitation/400 nm emission and 345 nm excitation/530 nm emission we found a strong inherent endogenous fluorescence (Fig. 4A & 4C) of the BioOss^{®} wafers which interfered strongly with the measurements based on fluorescence staining of alkaline phosphatase activity (ELF-97) or cell nuclei (DAPI stain). Nevertheless it is possible to see a specific effect of alkaline phosphatase activity induced by 10 nM rhBMP-2 in Fig. 4B & 4D.

### Conclusion

In conclusion the presented data have shown that it is possible to chemically functionalize hydroxyapatite ceramics and to immobilize proteins such as rhBMP-2 which can be released with half lives of 30-190 days. The biological function of the surface can then be either chemotactic or juxtacrine or both.

### Literature

[1] Tadic, D. & Epple, M. (2004) A thorough physicochemical characterisation of 14 calcium phosphate-based bone substitution materials in comparison to natural bone. Biomaterials, 25, 987-994.
[2] Hollemann, A.F. & Wiberg, E. (1964) Lehrbuch der Anorganischen Chemie, 57.-70. edn, pp. 252-253, Walter de Gruyter & Co., Berlin.
[3] Kim, J.Y., Fenton, R.R., & Hunter, B.A.K.B.J. (2000) Powder Diffraction Studies of Synthetic Calcium and Lead Apatites. Aust. J. Chem., 53, 679-686.
[4] Mathew, M. & Takagi, S. (2001) Structures of Biological Minerals in Dental Research. J. Res. Natl. Inst. Stand. Technol., 106, 1035-1044.
[5] McDowell, H., Gregory, T.M., & Brown, W.E. (1977) Solubility of Ca5(PO4)3OH in the system Ca(OH)2-H3PO4-H2O at 5, 15, 25 and 37 °C. J. Res. Natl. Bur. Standards, 81A, 273-281.
[6] Zahn, D. & Hochrein, O. (2005) Computational Study of Interfaces between Hydroxyapatite and Water. Phys. Chem. Chem. Phys., 5, 4004-4007.
[7] Revell, P.A., Hing, K.A., Tanner, S.M., & Bonfield, W. (2005) Osseointegration of Porous Hydroxyapatite. In Knochenersatzmaterialien und Wachstumsfaktoren. (Schnettler,R. & Markgraf,E., eds), pp. 28-30. Georg Thieme Verlag, Stuttgart, New York.
[8] Jennissen, H.P. (1999) Verfahren zur Immobilisierung von Mediatormolekülen auf anorganischen und metallischen Oberflächen", "Method for Immobilizing Mediator Molecules on Inorganic and Metal Implant Materials". PCT Patent WO9926674A2 *(Priority date Nov. 24, 1997),* pp. 1-29 (+ 5 Figs), Munich.
[9] Jennissen, H.P., Zumbrink, T., Chatzinikolaidou, M., & Steppuhn, J. (1999) Biocoating of Implants with Mediator Molecules: Surface Enhancement of Metals by Treatment with Chromosulfuric Acid. Materialwiss. Werkstofftech., 30, 838-845.
[10] Heimann, R.B., Graßmann, O., Zumbrink, T., & Jennissen, H.P. (2001) Biomimetic Processes During in vitro Leaching of Plasma-Sprayed Hydroxyapatite Coatings for Endoprosthetic Applications. Materialwiss. Werkstofftech., 32, 913-921.
[11] Weibrich, G., Trettin, R., Gnoth, S.H., Götz, H., Duschner, H., & Wagner, W. (2000) Bestimmung der Größe der spezifischen Oberfläche von Knochenersatzmaterialien mittels Gasadsorption. Mund Kiefer Gesichts Chir., 4, 148-152.
[12] Jennissen, H.P. (1995) Ubiquitin and the Enigma of Intracellular Protein Degradation. Eur J Biochem, 231, 1-30.
[13] Studier, F.W., Rosenberg, A.H., Dunn, J.J., & Dubendorff, J.W. (1990) Use of T7 RNA polymerase to direct expression of cloned genes. Methods Enzymol., 185, 60-89.
[14] Vallejo, L.F., Brokelmann, M., Marten, S., Trappe, S., Cabrera-Crespo, J., Hoffmann, A., Gross, G., Weich, H.A., & Rinas, U. (2002) Renaturation and purification of bone morphogenetic protein-2 produced as inclusion bodies in high-cell-density cultures of recombinant Escherichia coli. J Biotechnol., 94, 185-194.
[15] Wiemann, M., Rumpf, H.M., Bingmann, D., & Jennissen, H.P. (2001) The Binding of rhBMP-2 to the Receptors of viable MC3T3 Cells and the Question of Cooperativity. Materialwiss. Werkstofftech., 32, 931-936.
[16] Chatzinikolaidou, M., Laub, M., Rumpf, H.M., & Jennissen, H.P. (2002) Biocoating of Electropolished and Ultra-Hydrophilic Titanium and Cobalt Chromium Molybdenium Alloy Surfaces with Proteins. Materialwiss. Werkstofftech., 33, 720-727.
[17] Chatzinikolaidou, M., Zumbrink, T., & Jennissen, H.P. (2003) Stability of Surface-Enhanced Ultrahydrophilic Metals as a basis for Bioactive rhBMP-2 Surfaces. Materialwiss. Werkstofftech., 34, 1106-1112.
[18] Voggenreiter, G., Hartl, K., Chatzinikolaidou, M., Rumpf, H.M., & Jennissen, H.P. (2001) Assessment of the Biological Activity of Chemically Immobilized rhBMP-2 on Titianum surfaces in vivo. Materialwiss. Werkstofftech., 32, 942-948.

**Table 1 - Immobilization of ¹²⁵I-Ubiquitin on HAP-Wafers**

| Initial Ubiquitin Concentration [mg/ml] | Immobilized Ubiquitin | | | | | |
|---|---|---|---|---|---|---|
| | control (HAP) | | non-covalent (HAP-APS) | | covalent (HAP-APS-CDI) | |
| | [mg/g] | [ng/cm²] | [mg/g] | [ng/cm²] | [mg/g] | [ng/cm²] |
| 0.1 | 0.020 ± 0.003 | 0.026 ± 0.003 | 0.304 ± 0.044 | 0.382 ± 0.055 | 0.806 ± 0.151 | 1.011 ± 0.189 |
| 0.5 | 0.040 ± 0.003 | 0.051 ± 0.004 | 0.444 ± 0.034 | 0.557 ± 0.043 | 1.898 ± 0.390 | 2.381 ± 0.489 |
| 1.0 | 0.067 ± 0.004 | 0.084 ± 0.004 | 1.203 ± 0.094 | 1.509 ± 0.118 | 4.357 ± 0.665 | 5.467 ± 0.834 |
| 3.5 | | | 2.409 ± 0.352 | 3.022 ± 0.442 | 9.700 ± 1.265 | 12.170 ± 1.588 |

For immobilization of ubiquitin the washed wafers (modified and non-modified) were incubated for 15 hours at room temperature in a solution of 50 mM sodium phosphate pH 10.0 (buffer A) containing ubiquitin (0.1 - 3.5 mg/ml). The amount of bound ubiquitin is determined by using ¹²⁵I-CT-Ubiquitin (specific activity ca 10 µCi/mg). After incubation the wafers were rinsed 3 times in 5 ml buffer A and counted in a γ-counter. The adsorbed amounts are given per g wafer (surface area of 1g ≈ 80 m² ≈ 8 x 10⁵ cm²).

**Table 2 - Immobilization of ¹²⁵I-rhBMP-2 on HAP-Wafers**

| Initial rhBMP-2 Concentration [mg/ml] | Immobilized rhBMP-2 | | | | | |
|---|---|---|---|---|---|---|
| | control (HAP) | | non-covalent (HAP-APS) | | covalent (HAP-APS-CDI) | |
| | [mg/g] | [ng/cm²] | [mg/g] | [ng/cm²] | [mg/g] | [ng/cm²] |
| 0.1 | 0.042 ± 0.003 | 0.053 ± 0.003 | 0.185 ± 0.049 | 0.232 ± 0.061 | 0.351 ± 0.125 | 0.441 ± 0.156 |
| 0.2 | 0.072 ± 0.011 | 0.090 ± 0.013 | 0.370 ± 0.076 | 0.465 ± 0.095 | 0.722 ± 0.249 | 0.906 ± 0.313 |
| 0.3 | 0.111 ± 0.017 | 0.139 ± 0.021 | 0.588 ± 0.088 | 0.738 ± 0.110 | 1.134 ± 0.421 | 1.423 ± 0.528 |

For immobilization of rhBMP-2 the washed wafers (modified and non-modified) were incubated for 15 hours at room temperature in a solution of 125 mM borate, 5 mM CHAPS, 0.066 % SDS, pH 10.0 (buffer B) containing rhBMP-2 (0.1 - 0.3 mg/ml). The amount of bound rhBMP-2 is determined by using ¹²⁵I-CT-rhBMP-2 (specific activity ca 10 µCi/mg). After incubation, the wafers were rinsed 3 times in 5 ml buffer B and counted in a γ-counter. For further details see legend to Table1, Methods and the text.

**Table 3 - Dissociation rate constants obtained for the release of proteins from HAP-wafers**

| Protein | Surface | Initial Protein Load | Apparent first-order rate constants of dissociation | | Goodness of fit |
|---|---|---|---|---|---|
| | | [mg/g] | [Days⁻¹] | | [r²] |
| | | | k'₋₁ | k'₋₂ | |
| Ubiquitin | HAP (control) | 0.067 | 7.51 ± 0.47 | 9.36 x 10⁻² ± 3.71 x 10⁻³ | 0.99 |
| | HAP-APS | 1.2 | 5.42 x 10⁻¹ ± 4.81 x 10⁻² | 1.20 x 10⁻² ± 5.13 x 10⁻⁴ | 0.99 |
| | HAP-APS-CDI | 4.4 | monophasic | 4.55 x 10⁻³ ± 2.22 x 10⁻⁴ | 0.96 |
| rhBMP-2 | HAP (control) | 0.11 | 3.04 x 10⁻¹ ± 5.34 x 10⁻² | 3.17 x 10⁻² ± 1.91 x 10⁻³ | 0.99 |
| | HAP-APS | 0.59 | 7.20 x 10⁻¹ ± 1.96 x 10⁻¹ | 2.29 x 10⁻² ± 7.68 x 10⁻⁴ | 0.99 |
| | HAP-APS-CDI | 1.13 | 1.33 ± 0.39 | 3.73 x 10⁻³ ± 7.54 x 10⁻⁵ | 0.99 |

Unless otherwise stated the data were fitted to a two-phase exponential decay equation, The two apparent rate constants (k₋₁ for fast phase, k₋₂ for slow phase) are given. The number of data points (n) can be gathered from Figs 3 & 4 and ranged from 12-22. The half-lives can be calculated from the equation t_{1/2} = ln2/k' and are shown in Figs. 3 & 4. The conditions for obtaining the adsorption data can be taken from Tables 1 and 2. For further details see Methods and the text.

**Table 4 - Mean values for sustained release of ubiquitin and rhBMP-2 from Orthoss Spongiosa wafers**

| Protein | Surface | Initial Protein Load | Mean Sustained Release | Release Calculated for Duration of first Half-life (ln2/k'₋₂) | Total amount released |
|---|---|---|---|---|---|
| | | [mg/g] | [µg/g · day] | [days] | [µg/g] |
| Ubiquitin | HAP (control) | 0.067 | 1.9 | 7.4 | 33 |
| | HAP-APS | 1.2 | 10.0 | 57.6 | 600 |
| | HAP-APS-CDI | 4.4 | 14.5 | 152 | 2200 |
| rhBMP-2 | HAP (control) | 0.11 | 2.1 | 22 | 55 |
| | HAP-APS | 0.59 | 9.8 | 30 | 295 |
| | HAP-APS-CDI | 1.13 | 3.0 | 186 | 550 |

The mean values for sustained release were calculated in a simplified manner by dividing one half of the initial protein load by the number of days given by the half-life. In general the amount released by the first phase can be neglected. In the case of the ubiquitin and rhBMP-2 controls (HAP) the 39 and 19 µg released respectively in the first burst phase are not contained in the sustained release values but are found in the total amount released. The values are given per g wafer. For further details see Tables 3 & 4, Fig. 2 & 3, Methods and the text.

**Table 5 - Immobilization of ¹²⁵I-rhBMP-2 (E. coli) on Titanium SLA Surfaces in the presence of Conformational Enhancer (CHAPS)**

| **Titanium SLA-Surface Functionalization** | **Immobilized ¹²⁵I-rhBMP-2 c₀ = 0.15 mg/ml** | |
|---|---|---|
| | (E. coli, Univ. Essen) | |
| | **ng/cm²** | |
| | Classical Buffer System | Conformational Enhancer (CHAPS) |
| Control SLA | 10 ± 3 | - |
| CSA-surface (ultra-hydrophilic) | 9 ± 2 | 382 ± 74 |
| APS / Adsorption | 305 ± 79 | 2928 ± 546 |
| CDI / linkage | 257 ± 116 | 3652 ± 526 |

| | | |
|---|---|---|
| Number of experiments: n = 3 | | |

## Claims

1. Process for loading the surface of an implant with biologically active molecules whereby at least the loading step with said biologically active molecules is carried out in the presence of at least one tenside whereby said at least one tenside is bile acid or a bile acid derivative having a zwitterionic character and whereby said biologically active molecule is selected from growth factors of the class of TGF proteines, vascular growth factors, ubiquitin, antibiotics or mixtures thereof.

2. Process according to claim 1 whereby said bile acid derivative having a zwitterionic character is 3-[(3-Cholamidopropyl)-dimethylammonio]-propansulfonat (CHAPS).

3. Process according to any of claims 1 to 2, whereby said implant is a metallic implant, ceramic implant or a combination thereof.

4. Process according to claim 3, whereby said ceramic implant is comprising hydroxyapatite.

5. Process according to any of the preceding claims whereby said biologically active molecule is selected from growth factors of the class of BMPs, more preferably BMP-2, VEGF, angiotropin, or mixtures thereof.

6. Implant, obtainable according to the process according to any of the preceding claims.

7. Use of bile acid or a bile acid derivative having a zwitterionic character for enhancing the chemisorption of biologically active molecules on surfaces, in particular hydrophilic surfaces.

## Patentansprüche

1. Verfahren zum Beladen der Oberfläche eines Implantats mit biologisch aktiven Molekülen, wobei zumindest der Beladungsschritt mit den biologisch aktiven Molekülen in Gegenwart wenigstens eines Tensids ausgeführt wird, wobei das wenigstens eine Tensid Gallensäure oder ein Gallensäure-Derivat mit zwitterionischem Charakter ist und wobei das biologisch aktive Molekül aus Wachstumsfaktoren der Klasse der TGF-Proteine, Gefäßwachstumsfaktoren, Ubiquitin, Antibiotika oder Mischungen davon ausgewählt wird.

2. Verfahren nach Anspruch 1, wobei das Gallensäure-Derivat mit zwitterionischem Charakter 3-[(3-Choloamidopropyl)-dimethylammonio]-Propansulfonat (CHAPS) ist.

3. Verfahren nach einem der Ansprüche 1 bis 2, wobei das Implantat ein metallisches Implantat, keramisches Implantat oder eine Kombination davon ist.

4. Verfahren nach Anspruch 3, wobei das keramische Implantat Hydroxyapatit umfasst.

5. Verfahren nach einem der vorhergehenden Ansprüche, wobei das biologisch aktive Molekül aus Wachstumsfaktoren der Klasse der BMPs, bevorzugter BMP-2, VEGF, Angiotropin oder Mischungen davon ausgewählt wird.

6. Implantat, erhältlich nach dem Verfahren nach einem der vorhergehenden Ansprüche.

7. Die Verwendung von Gallensäure oder einem Gallensäure-Derivat mit zwitterionischem Charakter zur Steigung der Chemiesorption von biologisch aktiven Molekülen auf Oberflächen, insbesondere auf hydrophilen Oberflächen.

## Revendications

1. Processus pour charger la surface d'un implant avec des molécules biologiquement actives selon lequel au moins l'étape de chargement avec lesdites molécules biologiquement actives est effectuée en présence d'au moins un tensioactif selon lequel ledit au moins un tensioactif consiste en de l'acide biliaire ou en un dérivé de l'acide biliaire possédant un caractère zwitterionique et selon lequel ladite molécule uniquement active est choisie parmi des facteurs de croissance de la classe des protéines TGF, des facteurs de croissance vasculaire, de l'ubiquitine, des antibiotiques ou des mélanges de ceux-ci.

2. Processus selon la revendication 1 selon lequel ledit dérivé de l'acide biliaire possédant un caractère zwitterionique est le 3-[(3-cholamidopropyl)-diméthylammonio]-propansulfonat (CHAPS).

3. Processus selon l'une quelconque des revendications 1 à 2, selon lequel ledit implant est un implant métallique, un implant céramique ou une combinaison de ceux-ci.

4. Processus selon la revendication 3, selon lequel ledit implant céramique comprend de l'hydroxyapatite.

5. Processus selon l'une quelconque des revendications précédentes selon lequel ladite molécule biologiquement active est choisie parmi les facteurs de croissance de la classe des BMPs, plus préférablement BMP-2, VEGF, angiotropine, ou des mélanges de ceux-ci.

6. Implant, qui peut être obtenu par le processus selon l'une quelconque des revendications précédentes.

7. Utilisation d'acide biliaire ou d'un dérivé de l'acide biliaire possédant un caractère zwitterionique pour améliorer la chimisorpttion de molécules biologiquement actives sur des surfaces, en particulier des surfaces hydrophiles.
